# EUROPEAN PATENT APPLICATION

(11) **EP 1 783 211 A1**
(43) Date of publication of application: **09.05.2007**
(21) Application number: 04746550.5
(22) Date of filing: 22.06.2004
(51) Int. Cl.: C12N 7/00, C12Q 1/70, G01N 33/53

(54) **HBV PARTICLES CONTAINING HBV-RNA**

(71) Applicant: Advanced Life Science Institute, Inc., Wako-shi, Saitama 3510112 (JP)
(72) Inventor: MAKI, Noboru, Advanced Life Science Institute, Inc, Wako-shi, Saitama 351-0112 (JP); KIMURA, Tatsuji, Advanced Life Science Inst., Inc, Wako-shi, Saitama 351-0112 (JP); MATSUMOTO, Akihiro, Matsumoto-shi,Nagano 390-0861 (JP); ROKUHARA, Akinori, Matsumoto-shi, Nagano 390-0303 (JP); TANAKA, Eiji, Matsumoto-shi, Nagano 390-0312 (JP); KIYOSAWA, Kendo, Matsumoto-shi, Nagano 390-0305 (JP)
(74) Representative: Denison, Christopher Marcus
(86) International application number: PCT/JP2004/009081
(87) International publication number: WO 2005/123910

(57) **Abstract**

Hepatitis B virus (HBV) particles that contain HBV-RNA but do not contain HBV-DNA in a body fluid sample; A method of measuring the particles according to claim 1 by measuring HBV-RNA; A method of judging the therapeutic effect of the treatment of HBV infections by a drug having an effect of inhibiting RNA-dependent DNA polymerase, which method comprises performing the measurement on a body fluid of a patient who received said treatment; A method of predicting the risk of developing hepatic carcinoma or selecting risk groups of developing hepatic carcinoma by the above measurement.

## Description

### Field of the Invention

The present invention relates to virus particles comprising hepatitis B virus (HBV)-RNA which are useful for judging the therapeutic effects of therapeutic agents, such as lamivudine, on type B chronic hepatitis.

### Background Art

It is estimated that the number of people infected with hepatitis B virus (HBV) amounts to about 300 million throughout the world. HBV infection causes acute and chronic hepatitis (type B hepatitis), and furthermore cirrhosis and hepatic carcinoma.

As HBV particles in the serum of HBV-infected patients, there are particles comprising incomplete HBV-DNA and small globular particles and tubular particles composed of HBsAg in addition to Dane particles (complete HBV particles having infectivity). Furthermore, there are particles that comprise pre-core proteins as the structural protein but have no HBV-DNA (Japanese Patent Application No. 2002-261666). HBV particles of the present invention include all of these particles.

For the diagnosis of type B hepatitis, in addition to histological examination of liver, serum markers such as ALT, HBsAg, HBsAb, HBcAb, HBeAb, HBV polymerase and the amount of HBV-DNA are used. Among them, the amount of HBV-DNA in the serum is considered important for judging therapeutic effects of antiviral agents. Thus, when the amount of HBV-DNA is markedly decreased or below the detection limit, and this situation lasts for a long time with other indices being good, it is judged that a good therapeutic effect has been attained.

The gene of HBV is a circular incomplete double stranded DNA consisting of about 3,200 base pairs. The replication of the HBV gene comprises a step of reverse transcription. The replication process is roughly divided into the following four stages: (1) In the nucleus of the cell, complete double stranded circular DNA is formed by an endogenous DNA polymerase, which ring is then closed to form a covalently closed circular DNA (cccDNA). This cccDNA assumes an superhelical structure; (2) With cccDNA as the template, mRNA is transcribed by cellular RNA polymerase II. The longest one of 3.5 kb among them is pregenome RNA and serves as a template for reverse transcription; (3) An oligonucleotide is synthesized as a primer in the core particles by RNA-dependent DNA polymerase, and with the pregenome RNA as the template (-) strand DNA is synthesized by reverse transcription; and (4) With the oligonucleotide remaining at the 5'-end of the pregenome RNA as the primer and (-) strand DNA as the template, (+) strand DNA is synthesized.

Although the core protein forms nucleocapsid particles together with pregenome RNA, it was reported that it forms enveloped HBV particles containing HBsAg only after HBV-DNA was synthesized in the capsid as described above (Gerelsaikhan, T. et al., J. Virol. 70: 4269-4274, 1996). Thus, it was thought that there are no virus particles containing only HBV-RNA secreted outside the cell.

Su et al. have demonstrated the presence of HBV-RNA in the serum (Su, Q. et al., Clinical Cancer Research 7: 2005-2015, 2001). It is unknown how this HBV-RNA exists but, as there are HBV-RNA-positive cases even though virus markers are negative, in addition to the above finding, it is thought to be released not as capsid-packaged virus particles or small globular or tubular particles but in a state enveloped by cell membrane vesicles. It has also been reported by Miller et al. that HBV particles in the blood contain DNA-RNA hybrid molecules (Miller, R.H. et al., Virology 139: 53-63, 1984), suggesting the presence of virus particles that contain both of HBV-DNA and HBV-RNA.

By inhibiting any of the above processes of viral propagation, it is believed, the replication of the HBV gene can be inhibited and viral propagation can be suppressed. Reference will be made herein to lamivudine, a representative HBV therapeutic agent, but it should be noted that virus particles of the present invention are not related only to treatment with lamivudine. As HBV replication includes a process of reverse transcription, and human immunodeficiency virus (HIV) also requires reverse transcriptase for its propagation, screening of the inhibitors RNA-dependent DNA polymerase as a therapeutic agent has been attempted.

As lamivudine also specifically inhibits reverse transcription, also a replication process of human immunodeficiency virus (HIV), it was initially developed as a therapeutic agent for HIV. However, after it was demonstrated to have a propagation-inhibiting effect against HBV, its development as a therapeutic agent for type B hepatitis was started in 1992.

The action mechanism of lamivudine against HBV, though not fully elucidated, is thought to be as follows. Lamivudine is an antiviral agent of a nucleoside (cytidine) derivative, and is phosphorylated to a triphosphate derivative which is an active form in the cell. One of the mechanisms of inhibiting propagation of HBV of this agent is to inhibit the reverse transcription of pregenome RNA by inhibiting RNA-dependent DNA polymerase (reverse transcriptase).

Another possible mechanism is that lamivudine is incorporated into the viral DNA strand thereby arresting its extension.

Although lamivudine has been widely used as a therapeutic agent for HBV infections through such an action mechanism, there are cases in which the reduction in the amount of HBV-DNA in the blood is not observed by lamivudine therapy and subsequent development of resistant strains against lamivudine and hepatitis flare-up becomes problematic. Also, there are quite a few cases in which drug-resistant strains develop from among the cases in which the amount of HBV-DNA had decreased to less than the detection limit by lamivudine therapy, and problems remain in the judgment of the therapeutic effects based on the amount of HBV-DNA.

It is reported that although drugs, that have an effect of inhibiting the reverse transcription such as lamivudine etc., can significantly decrease the amount of HBV-DNA in the blood, cccDNA of HBV virus exists in the liver and the amount of this cccDNA does not significantly decrease (Locarnini, S. and C. Birch, J. Hepatol. 30: 536-550, 1999). Thus, the amount of HBV-DNA alone is not sufficient in judging the therapeutic effect of such drugs, and sometimes histological examinations by, for example, liver biopsy may be performed. However, liver biopsy is accompanied by severe pain, and patients are forced to endure a heavy burden.

Therefore, a marker that reflects the amount of HBV in the liver, by hematological testing, would be very useful for the judgment of therapeutic effect, and it would not only alleviate the burden of patients but enable a more effective treatment.

After research from the above viewpoints, the inventors have found, for the first time and contrary to previous reports, that there are virus particles that do not contain HBV-DNA but contain HBV-RNA in the blood. Furthermore, the inventors have developed a method of measuring this virus particle and have shown that the result of this measurement could provide a useful marker for the judgment of therapeutic effect.
Non-patent document 1
   Gerelsaikhan, T. et al., J. Virol. 70: 4269-4274, 1996
Non-patent document 2
   Su, Q. et al., Clinical Cancer Research 7: 2005-2015, 2001
Non-patent document 3
   Miller, R.H. et al., Virology 139: 53-63, 1984
Non-patent document 4
   Locarnini, S. and C. Birch, J. Hepatol. 30: 536-550, 1999

### Disclosure of the Invention

As described above, the amount of HBV-DNA in the serum has been considered important for the judgment of the therapeutic effect of drugs, but it is not sufficient as a judgment criteria.

Thus, it is an object of the present invention to provide virus particles that are useful for the judgment of a therapeutic effect of therapeutic agents having an effect of inhibiting reverse transcription, for type B hepatitis, such as lamivudine.

By measuring the virus particles, a novel diagnostic reagent that contributes to effective treatment of type B hepatitis can be developed.

The present invention provides HBV particles that are present in a body fluid such as blood and that contain HBV-RNA but do not contain HBV-DNA. The particles are different from HBV-RNA in the serum reported by Su et al. in that the former has the structural protein of HBV, and are different from virus particles that contain both of HBV-DNA and HBV-RNA in that the former does not contain HBV-DNA.

The present invention also provides a method of measuring this HBV particles.

Furthermore, the present invention provides a method of quantifying HBV particles by quantifying HBV-RNA using RT-PCR.

According to the present invention, the effects of enhancing the treatment results of type B hepatitis and of avoiding, as much as possible, the development of resistant strains and hepatitis flare-up can be expected.

The present invention relates to the method for judging a therapeutic effect by measuring HBV-RNA in the treatment of hepatitis B virus (HBV) infections by drugs as having an effect of inhibiting RNA-dependent DNA polymerase such as lamivudine.

The present invention also encompasses a method of simultaneously detecting HBV-DNA and HBV-RNA.

Furthermore, the present invention provides a method, or a diagnostic reagent and a diagnostic kit for detecting the above HBV-RNA. Best Mode for Carrying out the Invention

The present invention will now be explained in detail below.

The inventors of the present application have measured HBV-DNA and HBcAg in the serum of patients that received lamivudine treatment. As a result, it was found that the lamivudine treatment can completely eliminate HBV-DNA from the patient serum but that, in some specimens, HBcAg, though decreased, cannot be completely eliminated.

When HBV-RNA in these patient samples was measured, it was found to have a kinetics different from that of HBV-DNA. Thus, it was demonstrated that, in samples of patients that received lamivudine treatment, HBV-RNA and the structural protein of HBV are present but HBV-DNA is absent. Since RNase that digests RNA is present in large quantities in patient samples such as serum, it is thought that no HBV-RNA is present in the free form in the samples, and that it is forming incomplete virus particles with HBV structural proteins such as HBcAg and HBsAg.

The HBV particles that do not contain HBV-DNA but contain HBV-RNA and that are formed from HBV structural proteins are a novel substance. Measurement of the HBV particles is thought to be useful for judging the therapeutic effect of drugs that have an effect of inhibiting RNA-dependent DNA polymerase in the treatment of HBV infections. It is especially useful when the drug that has an effect of inhibiting RNA-dependent DNA polymerase is lamivudine. Furthermore, it is useful for predicting the risk of developing hepatic carcinoma or selecting groups at risk of developing hepatic carcinoma.

Any method that can measure HBV-RNA in test samples may be used in measuring the virus particles. Preferably, reverse transcription (RT)-PCR may be mentioned. In addition to measuring RNA in order to measure the virus particles, the HBV structural protein forming particles with HBV-RNA may be measured.

### Examples

The following examples illustrate the present invention but it should be noted that they do not limit the scope of the present invention in any way.

### Example 1. Measurement of the amount of HBV gene in the serum

### (A) Purification of HBV pregenome

In order to isolate the HBV gene in Dane particles (the infective HBV particle: the complete HBV particle), the HBV gene in a serum from a HBV patient was purified using High Pure Viral Nucleic Acid kit (Roche Diagnostics) in the following method. One hundred µl of the serum from a HBV patient who received lamivudine treatment was mixed with 100 µl of the serum from a normal healthy subject to prepare 200 µl of a serum sample. To the serum sample, 200 µl of a working solution {50 µl of poly(A) carrier RNA (4 mg/ml) and 2.5 ml of the binding buffer [6 M guanidine-HCl, 10 mM urea, 10 mM Tris-HCl (pH 4.4), 20% Triton X-100 (v/v)]} and 50 µl of Proteinase K (18 mg/ml) were added and mixed, and incubated at 72°C for 10 minutes.

One hundred µl of isopropanol and the sample were mixed, and the mixture was transferred into a High Pure filter tube. It was centrifuged at 8000 rpm for 1 minute, and after discarding the flowthrough, 500 µl of the inhibitor removal buffer [5 M guanidine-HCl, 20 mM Tris-HCl (pH 6.6)] was injected and then centrifuged at 8000 rpm for 1 minute. After adding 450 µl of the washing buffer [20 mM NaCl, 2 mM Tris-HCl (pH 7.5)], it was centrifuged at 8000 rpm for 1 minute. Then it was washed again in 450 µl of the washing buffer and was centrifuged at 13000 rpm for 10 seconds. Fifty µl of sterile water was added to the filter tube, which was centrifuged at 8000 rpm for 1 minute, and the flowthrough was collected to prepare a solution of the HBV gene in the serum.

### (B) Quantification of HBV-DNA in the serum

The quantification of HBV-DNA in the serum was performed by the TaqMan PCR method that employs the Light Cycler (Roche Diagnostics).

Real time PCR was performed in which 2 µl of 10× Light Cycler-FastStart DNA Master hybridization probe (Roche Diagnostics), 4 µl of the HBV gene solution, 2 µl each of two 10 µM primers corresponding to the S region of HBV [HBS-F: 5'-ACAACATCAGGATTCCTAGGAC-3' (SEQ ID NO: 1), HBS-R: 5'-GGTTGGTGAGTGATTGGAGGTT-3' (SEQ ID NO: 2)], 2 µl of 4 µM TaqMan probe [HBS-P: 5' FAM(6-carboxy-fluorescein)-CAGAGTCTAGACTCGTGGTGGACTTC-3' TAMRA(6-carboxy-tetramethyl-rhodamine) (SEQ ID NO: 3)] and 2 µl of 25 mM MgCl₂ were mixed, to which a sterile water was added to prepare a 20 µl of a reaction solution.

The condition included pretreatment at 95°C for 10 minutes, DNA denaturation at 95°C for 5 seconds, annealing at 60°C for 15 seconds and DNA synthesis at 72°C for 8 seconds, and fluorescence emitted from the TaqMan probe was measured to quantify the amount of HBV-DNA in the serum (Table 1).

### (C) Quantification of the HBV gene (DNA + RNA) in the serum

In order to measure the amount of HBV gene in the serum, the step of the reverse transcriptase reaction was added. To 10 µl of the HBV gene solution, 1 µl of 20 mM dNTP Mix (10 mM dATP, 10 mM dGTP, 10 mM dCTP, 10 mM dTTP) and 0.2 µl of 10 µM HBS-R primer were mixed, and after incubated at 65°C for 5 minutes, it was quickly cooled on ice. Four µl of 5 × reverse transcription buffer [250 mM Tris-HCl (pH 8.3), 375 mM KCl, 15 mM MgCl₂], 0.2 µl of 0.1 M DTT, 0.6 µl of RNase inhibitor (40 units/µl) and 3 µl of sterile water were mixed and then subjected to an annealing reaction at 42°C for 2 minutes, to which 1 µl of the reverse transcriptase (200 units/µl) was added to prepare a 20 µl of a reaction mixture, which was subjected to a reverse transcription reaction at 42°C for 50 minutes and the inactivation of the reverse transcriptase was performed at 70°C for 15 minutes.

By applying 4 µl of this reaction mixture to the above HBV-DNA quantification system, the amount of the HBV gene in the serum, i.e. the total amount of HBV-DNA and RNA in the serum, can be measured. Also, by subtracting the amount of HBV-DNA from the amount of the HBV gene, the amount of HBV-RNA in the serum can be estimated (Table 1).

### Example 2. Measurement of the amount of HBc antigen

### (A) Preparation of an antibody-immobilized plate

Three monoclonal antibodies HB44 (recognition site: amino acids 31-49), HB61 (recognition site: amino acids 131-140) and HB114 (recognition site: amino acids 1-81) that react both HBc antigen and HBe antigen were coated and immobilized to a microtiter plate. After washing with PBS, it was blocked with a solution containing casein and, after removing the solution, it was dried.

### (B) Pretreatment of samples

Fifty µl of the pretreatment solution (15% sodium dodecyl sulfate [SDS], 3% CHAPS, 1% hexadecyltrimethyl ammonium bromide) was mixed with 100 µl of the sample (serum, plasma etc.) and treated at 70°C for 30 minutes.

### (C) Primary reaction

To each antibody-immobilized well, 100 µl of the reaction buffer (pH 8.0) and 50 µl of the pretreated sample were added, and the plate was reacted under gentle stirring at room temperature for 2 hours.

### (D) Secondary reaction

After washing the wells, 100 µl of a solution containing alkaline phosphatase-labelled HB50 (recognition site: amino acids 168-176) monoclonal antibody was added to each well, and the plate was incubated at room temperature for 1 hour.

### (E) Substrate reaction

After washing the wells, 100 µl of the CDP Star with Emerald II (Applied Biosystems) was added, and the intensity of luminescence after reacting at room temperature for 20 minutes was measured using a microtiter plate luminometer. It was compared to the intensity of luminescence of the standard antigen and the concentration of HBc antigen in the sample was calculated (Table 1).

### Example 3. Investigation with the serum from HBV patients who received lamivudine treatment

As shown in Table 1, in a sample taken one month before the start of the lamivudine treatment, the amounts of HBV-DNA and RNA in the blood were nearly equal whereas in a sample after administration of the lamivudine for one month, the concentration of HBV-DNA in the blood was 6.1 × 10³ copies/ml whereas that of HBV-RNA in the blood was 1.0 × 10⁵ copies/ml, clearly indicating that the amount of HBV-RNA in the blood was dominant. Furthermore, in samples after 8 months of administration and after 19 months of administration, HBV-RNA at the concentration of 2 to 7 × 10³ copies/ml was present in the blood even though HBV-DNA in the blood was practically undetectable.

On the other hand, the concentration of HBc antigen before the administration was 610 pg/ml, whereas one month after the administration it decreased to 56 pg/ml. But the rate of reduction in HBc antigen was smaller than that in HBV-DNA, and the concentration of HBc antigen was 21 and 20 pg/ml at 8 and 19 months after administration, respectively, indicating that the reduction of HBc antigen was hardly observed. Changes in concentration of HBc antigen were almost identical to those in the amount of (HBV-DNA + HBV-RNA).

These results suggested that, in the serum from patients under lamivudine treatment, substantially no complete virus particles containing HBV-DNA are present, and HBV particles containing HBV-RNA alone are present in large quantities.

**Table 1**

| Serum from HBV patient who received lamivudine treatment | Amount of HBV-DNA (copies/ml) | Amount of HBV gene (copies/ml) | Amount of HBV-RNA (copies/ml) | Amount of HBc antigen (pg/ml) |
|---|---|---|---|---|
| One month before administration | 2.4×10⁶ | 7.8×10⁶ | 5.4×10⁶ | 610 |
| One month after administration | 6.1×10³ | 1.1×10⁵ | 1.0×10⁵ | 56 |
| 8 months after administration | 1.0×10² | 7.2×10³ | 7.1×10³ | 21 |
| 19 months after administration | 0 | 2.2×10³ | 2.2×10³ | 20 |

## Claims

1. Hepatitis B virus (HBV) particles that contain HBV-RNA but do not contain HBV-DNA in a body fluid sample.

2. A method of measuring the particles according to claim 1 by measuring HBV-RNA.

3. A method of measuring the particles according to claim 1 by the RT-PCR method.

4. A method of judging the therapeutic effect of the treatment by a drug having an effect of inhibiting RNA-dependent DNA polymerase of HBV infections, which method comprises performing the measurement according to claim 2 or 3 on a body fluid of a patient who received said treatment.

5. The method according to claim 4 wherein said drug having an effect of inhibiting RNA-dependent DNA polymerase is lamivudine.

6. A method of predicting the risk of developing hepatic carcinoma or selecting risk groups of developing hepatic carcinoma by the measurement according to claim 2 or 3.


SEQUENCE LISTING
<110>Applied Life Science Institute, Inc.
<120>HBV particles containing HBV-RNA
<130>P813
<160>3
<210>1
<211>22
<212>DNA
<213>Artificial  Sequence
<220>
<221>
<222>
<223>Primer
<400>1
acaacatcag gattcctagg ac                                                    22
<210>2
<211>22
<212>DNA
<213>Artificial  Sequence
<220>
<221>
<222>
<223>Primer
<400>2
ggttggtgag tgattggagg tt                                                    22
<210>3
<211>26
<212>DNA
<213>Artificial  Sequence
<220>
<221>
<222>
<223>Probe
<400>3
cagagtctag actcgtggtg gacttc                                                26